# EUROPEAN PATENT APPLICATION

(11) **EP 2 664 604 A1**
(43) Date of publication of application: **20.11.2013**
(21) Application number: 12734568.4
(22) Date of filing: 11.01.2012
(51) Int. Cl.: C07C 7/04, C07C 11/02

(54) **PROCESS FOR PRODUCING OLEFIN OLIGOMER MIXTURE**

(30) Priority: 13.01.2011 JP 2011004943
(71) Applicant: Idemitsu Kosan Co., Ltd., Chiyoda-ku Tokyo 100-8321 (JP)
(72) Inventor: YOKOTA, Tatsuyoshi, Ichihara-shi Chiba 299-0193 (JP); UCHIYAMA, Shoichi, Ichihara-shi Chiba 299-0193 (JP); SATO, Hideki, Ichihara-shi Chiba 299-0193 (JP); MIYAMOTO, Shinji, Ichihara-shi Chiba 299-0193 (JP)
(74) Representative: Vossius & Partner
(86) International application number: PCT/JP2012/000099
(87) International publication number: WO 2012/096159

(57) **Abstract**

A method for producing an olefin oligomer mixture comprising: feeding a raw olefin oligomer mixture to a first evaporator; feeding a residue which is taken out from the first evaporator to a second evaporator, and returning a distillate which is distilled from the second evaporator to the first evaporator; taking a distillate out from the first evaporator; feeding a residue which is taken out from the second evaporator to a third evaporator; and taking a distillate out from the third evaporator.

## Description

### Technical Field

The invention relates to a method for producing an olefin oligomer mixture and an olefin oligomer mixture obtained by this method. In particular, the invention relates to a method for separating an olefin oligomer mixture having a specific viscosity from an olefin oligomer reaction product obtained by polymerizing an α-olefin monomer.

### Background Art

An oligomer of α-olefin having 6 to 20 carbon atoms is produced mainly as a raw material oil of synthetic lubricant oil such as engine oil. Of α-olefin oligomers, synthetic lubricant oil containing an oligomer of 1-decene and an oligomer of 1-octene and 1-dodecene as a main component is significantly useful as raw material oil for high-performance engine oil due to its low viscosity. Therefore, its demand has been increasing. The important property as the base for lubricant oil is kinetick viscosity. Application of the lubricant oil depends on kinetick viscosity, and hence, it is necessary to conduct separation according to kinetick viscosity.

An oligomer reaction product is produced by polymerizing an α-olefin as a monomer. Therefore, an oligomer reaction product is obtained as a mixture of a plurality of oligomers differing in polymerization degree (for example, a trimer, a tetramer, a pentamer or the like). Therefore, synthetic lubricant oils (oligomer mixture) which are obtained according to viscosity have a problem that lubricant oils have different properties due to difference in composition of oligomers even if they have the same viscosity.

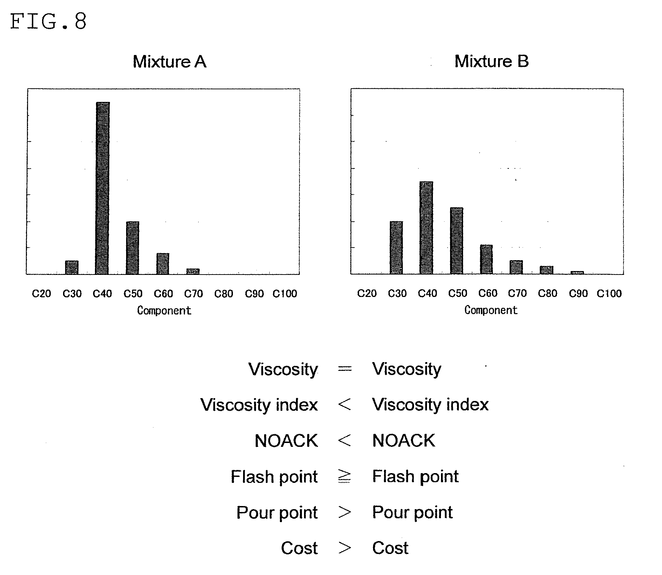

For example, the composition distributions of oligomer mixtures A and B are shown in FIG. 8. CX0 indicates an X-mer. For example, C30 indicates a trimer, C40 indicates a tetramer and C50 indicates a pentamer. The vertical axis indicates the content thereof. Mixture A and mixture B have the same viscosity. However, as compared with mixture B, mixture A contains C30 and C50 in a relatively smaller amount than that of C40 (i.e. composition distribution is narrow). As a result, as shown in FIG. 8, mixture A and mixture B differ in properties such as viscosity index, NOACK (the ratio (wt%) of evaporation at 250°C for 1 hour), flash point and pour point or cost.

In order to supply as a product, it is required to adjust not only viscosity but also properties. Even if the viscosity is the same, it is necessary to adjust the composition of oligomers in accordance with the required properties. In particular, in order to stabilize the quality, an oligomer mixture is required to have a low NOACK.

Patent Document 1 discloses a method for separating a trimer of α-olefin by a hydrogenation treatment and a steam treatment.

### Related Art Documents

### Patent Document

Patent Document 1: JP-A-H05-201884

### Summary of the Invention

An object of the invention is to provide a method for obtaining an olefin oligomer mixture with a specific viscosity from an olefin oligomer mixture by controlling the composition of oligomers.
An object of the invention is to provide an olefin oligomer mixture having a specific composition(composition distribution).

According to the invention, the following method for producing an olefin oligomer mixture or the like is provided.
1. A method for producing an olefin oligomer mixture comprising:
   feeding a raw olefin oligomer mixture to a first evaporator;
   feeding a residue which is taken out from the first evaporator to a second evaporator, and returning a distillate which is distilled from the second evaporator to the first evaporator;
   taking a distillate out from the first evaporator;
   feeding a residue which is taken out from the second evaporator to a third evaporator; and
   taking a distillate out from the third evaporator.
2. A method for producing an olefin oligomer mixture comprising:
   feeding a raw material olefin oligomer mixture to a first evaporator;
   feeding a residue which is taken out from the first evaporator to a second evaporator, and returning a distillate which is distilled from the second evaporator to the first evaporator;
   feeding a residue which is taken out from the second evaporator to a third evaporator;
   taking a distillate out from the third evaporator;
   feeding a distillate which is distilled from the first evaporator to a fourth evaporator and returning a residue which is taken out from the fourth evaporator to the first evaporator; and
   taking a distillate out from the fourth evaporator.
3. The method according to 2, further comprising:
   connecting in series m (m is an integer of 1 or more) evaporators between the first evaporator and the fourth evaporator;
   feeding to them evaporators distillates which are distilled from upstream evaporators thereof, respectively; and
   returning residues which are taken out from the m evaporators to upstream evaporators thereof, respectively.
4. The method according to 3, wherein m is 1 or 2.
5. The method according to any of 1 to 4, further comprising:
   connecting in series n (n is an integer of 1 or more) evaporators between the second evaporator and the third evaporator;
   feeding to the n evaporators residues which are taken out from upstream evaporators thereof, respectively; and
   returning distillates which are distilled from the n evaporators to the upstream evaporators thereof, respectively.
6. The method according to 5, wherein n is 1 or 2.
7. The method according to any of 1 to 6, wherein the evaporator has a built-in condenser.
8. A distillate which is distilled from the first evaporator or the fourth evaporator according to the method according to any of 1 to 7.
9. A distillate which is distilled from the third evaporator according to the method according to any of 1 to 7.
10. An olefin oligomer mixture comprising 0.5 wt% or less of olefin oligomers having 20 or less carbon atoms and 9 to 12 wt% of olefin oligomers having 40 or more carbon atoms.
11. The olefin oligomer mixture according to 10 which has a kinetic viscosity at 100°C of 4 mm²/s or less.
12. An olefin oligomer mixture comprising 25 wt% or less of olefin oligomers having 30 or less carbon atoms and 14 to 50 wt% of olefin oligomers having 60 or more carbon atoms.
13. The olefin oligomer mixture according to 12 which has a kinetic viscosity at 100°C of 5.0 to 6.5 mm²/s.

According to the invention, it is possible to provide a method for obtaining an olefin oligomer mixture having a specific viscosity from an olefin oligomer mixture by controlling the composition of oligomers.
According to the invention, it is possible to provide an olefin oligomer mixture having a specific composition (composition distribution).

### Brief Description of the Drawings

FIG. 1 is a view showing embodiment 1 of the invention;
FIG. 2 is a view showing embodiment 2 of the invention;
FIG. 3 is a view showing embodiment 3 of the invention;
FIG. 4 is a view showing a standard method for separating an oligomer mixture having a specific viscosity from a raw oligomer mixture by means of evaporators;
FIG. 5 is a view showing the composition distribution of distillate D3 obtained in embodiment 1 and the composition distribution of distillate D3 obtained by the standard method;
FIG. 6 is a view showing the composition distribution of distillate D3 obtained in embodiment 2 and the composition distribution of distillate D3 obtained by the standard method;
FIG. 7 is a view showing the composition distribution of distillate D3 obtained in embodiment 3 and the composition distribution of distillate D3 obtained by the standard method; and
FIG. 8 is a view showing the relationship between the composition distribution and properties of an oligomer mixture.

### Mode for Carrying out the Invention

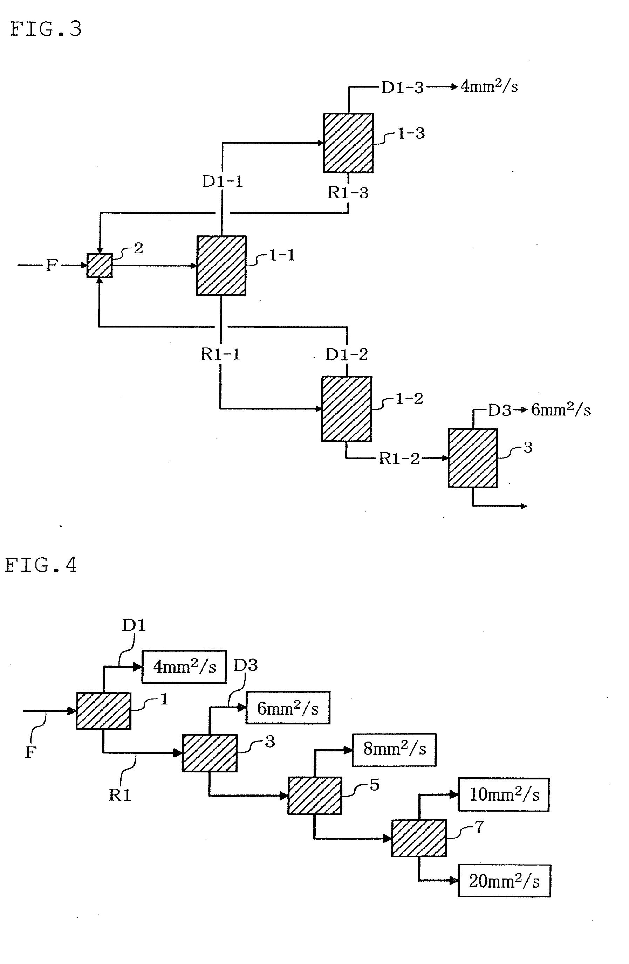

FIG. 4 shows a method for separating an oligomer mixture having a specific viscosity from a raw oligomer mixture by means of evaporators (hereinafter referred to as the "standard method"). Hereinbelow, an oligomer mixture with a specific viscosity which has been separated is referred to as the "oligomer component" in order to distinguish it from a raw oligomer mixture.

In FIG. 4, a raw oligomer mixture F is feeded to evaporator 1. From evaporator 1, distillate D1 which has the lowest viscosity (4 mm²/s, for example) is taken out. Residue R1 is feeded to evaporator 3. From evaporator 3, distillate D3 which has the second lowest viscosity (6 mm²/s, for example) is taken out. Further, a residue is feeded to evaporator 5. From evaporator 5, a distillate which has the third lowest viscosity (8 mm²/s, for example) is taken out. A residue is feeded to evaporator 7. From evaporator 7, a distillate which has the fourth lowest viscosity (10 mm²/s) is taken out. A residue (20 mm²/s, for example) is subjected to a subsequent treatment. In this way, by using four evaporators, oligomer components differing in viscosity can be separated.

However, for this method, due to the variation in composition of the oligomer mixture F feeded to evaporator 1, the composition of the oligomer components obtained with different viscosities are also varied. Further, as in mixture B shown in FIG. 8, low-viscous oligomers, middle viscous oligomers and high-viscous oligomers are respectively contained in a relatively large amount (i.e. composition distribution is wide).

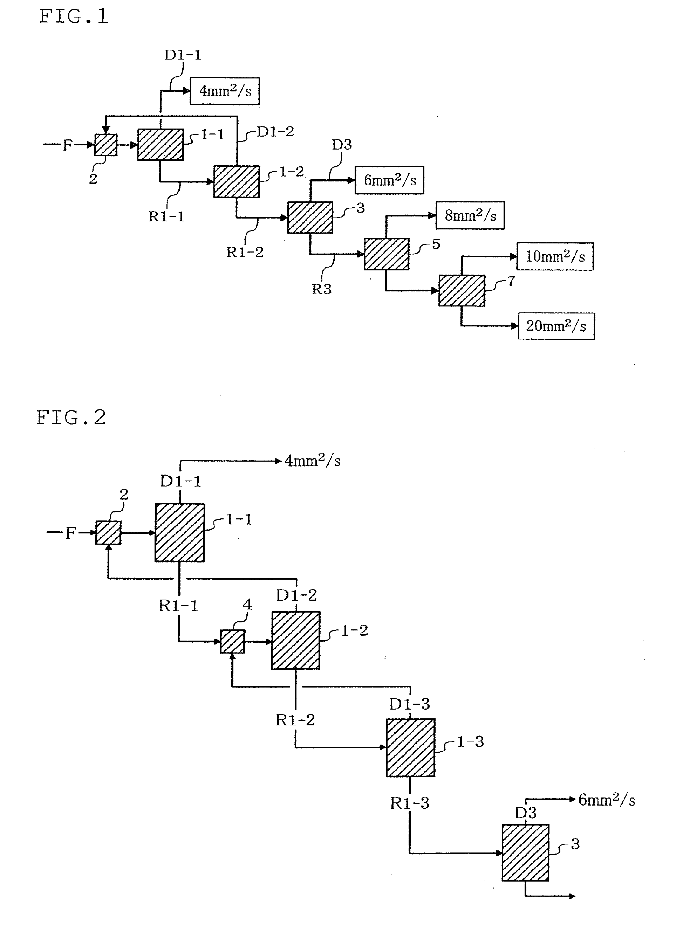

Next, one embodiment (embodiment 1) of the method of the invention will be explained with reference to FIG. 1.
Raw oligomer mixture F is feeded to evaporator 1-1 (first evaporator). Distillate D1-1 obtained from evaporator 1-1 serves as a product as it is. The remaining residue R1-1 is feeded to evaporator 1-2 (second evaporator). Distillate D1-2 obtained from evaporator 1-2 is feeded again to the evaporator 1-1 together with raw oligomer mixture F through a mixing device (a mixer or the like) 2. Residue R1-2 removed from evaporator 1-2 is feeded to evaporator 3 (third evaporator) and distillate D3 obtained from evaporator 3 serves as a product as it is. Residue R3 which has been taken out is feeded to evaporator 5. The subsequent processes are the same as those in the above-mentioned standard method. Light components D1-1 have been removed, and distillate D1-2 contains no heavy components R1-2. Therefore, as compared with raw material oligomer mixture F, distillate D1-2 contains relatively small amounts of light components D1-1 and heavy components R1-2, and contains a large content of middle components (it becomes a distillate having a narrow composition distribution having small amounts of light components and heavy components of the raw oligomer). Such recycling component D1-2 is returned to evaporator 1-1. As a result, the amount of low-viscosity components of residue R1-2 from evaporator 1-2 is decreased, whereby distillates obtained after distillate of D3 become components with a narrow composition distribution.

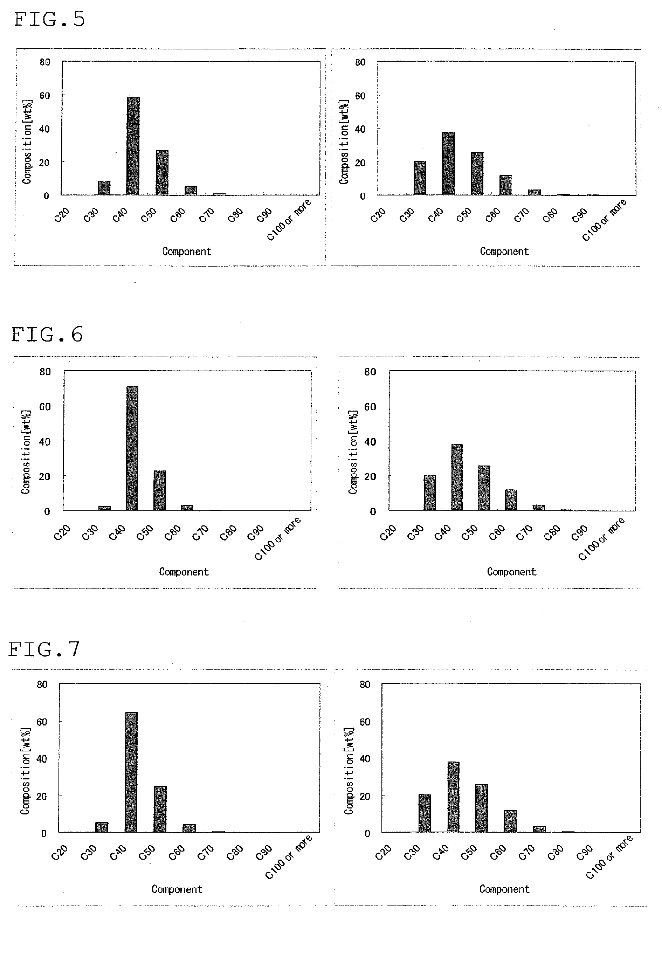

According to the method of this embodiment, the composition distribution of distillates obtained after distillate D3 becomes narrow. For example, FIG. 5 shows the composition distribution of distillate D3 obtained by the method of embodiment 1 and the composition distribution of distillate D3 obtained by the standard method. In FIG. 5, the left side of the figure shows the distillate obtained in embodiment 1 and the right side shows the distillate obtained by the standard method.

In embodiment 1, low-viscosity component D3 is obtained by using two evaporators (2 stages) in addition to evaporator 1-1. Low-viscosity component D3 may be obtained by using n + 2 evaporators (n + 2 stages) (n is an integer of 1 or more). n evaporators may be provided in series and/or in parallel relative to evaporator 1-1.
Although the composition distribution becomes sharp with an increase in number of stages, the equipment cost becomes high. A preferable number of stages is 3 or more, more preferably 4.

If the evaporators are provided in series, n evaporators are connected in series between evaporator 1-2 and evaporator 3. Residues which have been taken out from upstream (normally, immediately before) evaporators are respectively feeded to the n evaporators, and distillates which have been distilled from the n evaporators are respectively returned to upstream evaporators (evaporator 1-1 or other upstream evaporators).

Another embodiment (embodiment 2) of the invention will be explained with reference to FIG. 2. In this embodiment, one (n=1) evaporator is connected in series between evaporator 1-2 and evaporator 3, whereby low-viscosity component D3 is obtained by three stages.
To the evaporator 1-1, raw oligomer mixture F is feeded to a mixing device 2 (a mixer or the like). Residue R1-1 is taken out from the evaporator 1-1, and is feeded to evaporator 1-2 via mixer 4.
Distillate D1-2 is taken out from evaporator 1-2, and is feeded to evaporator 1-1 via mixer 2. Residue R1-2 is taken out from evaporator 1-2, and is feeded to evaporator 1-3.
Distillate D1-3 is taken out from evaporator 1-3, and is feeded to evaporator 1-2 via mixer 4. Residue R1-3 is taken out from evaporator 1-3 and is feeded to evaporator 3, and distillate D3 obtained from evaporator 3 serves as a product as it is. The processes subsequent to the evaporation by evaporator 3 are the same as those in the above-mentioned standard method.

As for the return of a distillate to an upstream evaporator, as shown in FIG. 2, a distillate may be returned to the nearest evaporator or may be returned to a distant evaporator. For example, residue D1-3 of the evaporator 1-3 may be returned to evaporator 1-1, not evaporator 1-2.

According to the method of this embodiment, the composition distributions of distillates obtained after distillate D3 become narrow. For example, FIG. 6 shows the composition distribution of distillate D3 obtained by the method of embodiment 2 and the composition distribution of distillate D3 obtained by the standard method. In FIG. 6, the left side of the figure shows composition distribution of distillate obtained in embodiment 2 and the right side of the figure shows composition distribution of distillate obtained by the standard method.

Still another embodiment (embodiment 3) of the invention will be explained with reference to FIG. 3. In this embodiment, one (n=1) evaporator (the fourth evaporator) is connected to evaporator 1-1 in parallel with evaporator 1-2, whereby low-viscosity component is obtained by three stages.
Raw oligomer mixture F is feeded to evaporator 1-1 through mixer 2. Residue R1-1 is taken out from evaporator 1-1 and is feeded to evaporator 1-2, and distillate D1-1 is taken out from evaporator 1-1 and is feeded to evaporator 1-3.
Residue R1-3 is taken out from evaporator 1-3, and is feeded to evaporator 1-1 via mixer 2.
Distillate D1-2 is taken out from evaporator 1-2, and is feeded to evaporator 1-1 via mixer 2. Residue R1-2 is taken out from evaporator 1-2 and is feeded to evaporator 3. Distillate D3 obtained from evaporator 3 serves as a product as it is. The processes subsequent to the evaporation by evaporator 3 are the same as those of the above-mentioned standard method.

According to the method of this embodiment, the composition distribution of the distillate obtained after distillate D3 becomes narrow. For example, FIG. 7 shows the composition distribution of distillate D3 obtained by the method of embodiment 3 and the composition distribution of distillate D3 obtained by the standard method. In FIG. 7, the left side of the figure shows the composition distribution of distillate obtained in embodiment 3 and the right side shows the composition distribution of distillate obtained by the standard method.

The number of stages of pararell recycling of the embodiment can be increased as in the case of connection in series. For example, as mentioned above, n evaporators are connected in series between evaporator 1-2 and evaporator 3. To the n evaporators, residues taken out from upstream evaporators are feeded, respectively, and residues taken out from the n evaporators are returned to upstream evaporators, respectively. Similarly, m evaporators (m is an integer of 1 or more) are connected in series between evaporator 1-1 and evaporator 1-3. To the m evaporators, distillates which have been distilled from upstream evaporators are feeded, respectively, and residues taken out from the m evaporators are returned to upstream evaporators, respectively.

In the above-mentioned embodiment, recycle components are feeded in the form of a mixture of a raw material to be feeded to an upstream evaporator. However, the recycle components may be directly returned to an upstream evaporator.

Embodiment 2 is an example of series recycling (return), and embodiment 3 is an example of countercurrent (parallel) recycling (return). In the case of series recycling, the heat duty required in the second evaporator is larger than the heat duty required in the first evaporator (the same applies to the subsequent stages). Therefore, no unnecessary heat duty is generated, and hence, utility load is small. In the case of countercurrent recycling, the heat duty required in the second evaporator on the distillate side is smaller than the heat duty required in the first evaporator (the same applies to the subsequent stages). Accordingly, a distillate of the first evaporator which has been condensed by a condenser is heated again, resulting in generation of unnecessary heat duty. As a result, utility load becomes large.

The evaporator used in the method of the invention is preferably an evaporator with an internal condenser (short-path evaporator) since it can conduct distillation in the vacuum state without increasing temperature and hence decomposition of oligomers can be prevented.

In the above-mentioned embodiment, the kinetic viscosity at 100°C is 4 to 10 mm²/s. However, the kinetic viscosity is not limited thereto.

The oligomer mixture to be feeded to the evaporator 1-1 may be any type of a mixture as long as it comprises a plurality of oligomers. It may be a product by oligomerization containing a dimer, which is obtained by an oligomerization reaction of an α-olefin, or may be an oligomer mixture obtained by removing a dimer from this product by oligomerization by a method such as distillation.

In the method of the invention, a reaction product obtained by oligomerization reaction of an olefin having 6 to 18 carbon atoms is suited as the raw material. An olefin used for oligomerization may be an α-olefin or an inner olefin as long as it is an olefin having 6 to 18 carbon atoms. An α-olefin having 8 to 12 carbon atoms, in particular an α-olefin having 10 carbon atoms (1-decene), is preferable.

By using the above-mentioned method of the invention, an oligomer mixture of which the amount of an oligomer having a prescribed polymerization degree is larger than the amount of oligomers having other polymerization degrees (having lower and higher viscosities) is obtained. As a result, an oligomer mixture with lower NOACK and better practical properties in spite of similar kinetic viscosity can be obtained.

Specifically, an oligomer mixture having 0.5 wt% or less of oligomers having 20 or less carbon atoms and 9 wt% or more and 12 wt% or less of oligomers having 40 or more carbon atoms can be obtained as follows: An oligomer is produced by methods disclosed in JP-A-2001-335607, WO2010/074233 or Japanese Patent Application No. 2010-121937 or the like. A dimer and a monomer in a reaction product obtained by these methods are removed in advance by distillation, and purified by the above-mentioned method of the invention. It is preferred that this mixture have a kinetic viscosity at 100°C of 4 mm²/s or less. Further, an oligomer mixture containing 25 wt% or less of oligomers having 30 or less carbon atoms and containing 14 wt% or more and 50 wt% or less of oligomers having 60 or more carbon atoms can be obtained. It is preferred that this mixture have a kinetic viscosity at 100°C of 5.0 to 6.5 mm²/s or less. These mixtures obtained by the method of the invention have a narrow composition distribution and a low NOACK (4.5 to 5.0 wt%, the measuring method of NOACK: 250°C, 1 hour (ASTM D 5800)).

The composition distribution of an oligomer having a specific viscosity can be analyzed by gas chromatography. The kinetic viscosity at 100°C can be measured by means of a capillary kinetick viscometer.

### [Gas chromatography measurement conditions]

Column: HT-SIMDST (5 m × 0.53 mm × 0.17 µm)
Carrier flow: 40 cm/sec
Injection mode: Cool on-column injection
Injection and detection temperature: 440°C
Column temperature: 50°C (hold for 0.1 min), elevated at a rate of 20°C/min, 430°C (hold for 15 min)
INJ amount: 0.5 µL

### Sample concentration: 1 mass% toluene solution (containing 1 mass% of hexadecane internal standard)

In order to suppress thermal decomposition of a high-boiling oligomer during the production process of an olefin oligomer, the temperature of a fluid (oligomer mixture) is set to preferably 260°C or less, more preferably 220°C or less and further preferably 200°C or less. For that purpose, it is preferred that the temperature of a heating medium be set to 290°C or less. Some oligomers cannot be separated at 260°C. For such oligomers, the upper limit of the temperature is 280°C. The degree of decomposition can be measured using a bromine number as evaluation standards. However, if the temperature is too low, the viscosity is increased to deteriorate heat transmission, resulting in the need of pump power. Therefore, the temperature is appropriately adjusted.
Production processes of an olefin oligomer include polymerization of an olefin, deactivation of a catalyst, separation by distillation of an oligomer having a specific polymerization degree, hydrogenation of an oligomer having a specific polymerization degree, and the above-mentioned method of the invention.

### INDUSTRIALAPPLICABILITY

The method of the invention can be used for the production of raw material oil of synthetic lubricant oil.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.
The documents described in the specification are incorporated herein by reference in its entirety.

## Claims

1. A method for producing an olefin oligomer mixture comprising:
feeding a raw olefin oligomer mixture to a first evaporator;
feeding a residue which is taken out from the first evaporator to a second evaporator, and returning a distillate which is distilled from the second evaporator to the first evaporator;
taking a distillate out from the first evaporator;
feeding a residue which is taken out from the second evaporator to a third evaporator; and
taking a distillate out from the third evaporator.

2. A method for producing an olefin oligomer mixture comprising:
feeding a raw material olefin oligomer mixture to a first evaporator;
feeding a residue which is taken out from the first evaporator to a second evaporator, and returning a distillate which is distilled from the second evaporator to the first evaporator;
feeding a residue which is taken out from the second evaporator to a third evaporator;
taking a distillate out from the third evaporator;
feeding a distillate which is distilled from the first evaporator to a fourth evaporator and returning a residue which is taken out from the fourth evaporator to the first evaporator; and
taking a distillate out from the fourth evaporator.

3. The method according to claim 2, further comprising:
connecting in series m (m is an integer of 1 or more) evaporators between the first evaporator and the fourth evaporator;
feeding to the m evaporators distillates which are distilled from upstream evaporators thereof, respectively; and
returning residues which are taken out from the m evaporators to upstream evaporators thereof, respectively.

4. The method according to claim 3, wherein m is 1 or 2.

5. The method according to any of claims 1 to 4, further comprising:
connecting in series n (n is an integer of 1 or more) evaporators between the second evaporator and the third evaporator;
feeding to the n evaporators residues which are taken out from upstream evaporators thereof, respectively; and
returning distillates which are distilled from the n evaporators to the upstream evaporators thereof, respectively.

6. The method according to claim 5, wherein n is 1 or 2.

7. The method according to any of claims 1 to 6, wherein the evaporator has a built-in condenser.

8. A distillate which is distilled from the first evaporator or the fourth evaporator according to the method according to any of claims 1 to 7.

9. A distillate which is distilled from the third evaporator according to the method according to any of claims 1 to 7.

10. An olefin oligomer mixture comprising 0.5 wt% or less of olefin oligomers having 20 or less carbon atoms and 9 to 12 wt% of olefin oligomers having 40 or more carbon atoms.

11. The olefin oligomer mixture according to claim 10 which has a kinetic viscosity at 100°C of 4 mm²/s or less.

12. An olefin oligomer mixture comprising 25 wt% or less of olefin oligomers having 30 or less carbon atoms and 14 to 50 wt% of olefin oligomers having 60 or more carbon atoms.

13. The olefin oligomer mixture according to claim 12 which has a kinetic viscosity at 100°C of 5.0 to 6.5 mm²/s.
